(19) Europäisches Patentamt
European Patent Office
Office européen des brevets

(11) EP 4 429 764 B1

(12) EUROPEAN PATENT SPECIFICATION

(45) Date of publication and mention of the grant of the patent:
**22.07.2026 Bulletin 2026/30**

(21) Application number: **22823204.7**

(22) Date of filing: **07.11.2022**

(51) International Patent Classification (IPC):
***A61P 25/04*** $^{(2006.01)}$ ***C07D 239/47*** $^{(2006.01)}$
***A61K 31/505*** $^{(2006.01)}$

(52) Cooperative Patent Classification (CPC):
**C07D 239/47; A61P 25/04**

(86) International application number:
**PCT/US2022/049131**

(87) International publication number:
**WO 2023/081463 (11.05.2023 Gazette 2023/19)**

# (54) DEUTERATED (TRIFLUOROMETHYL)PYRIMIDINE-2-AMINE COMPOUNDS AS POTENTIATORS OF THE HMRGX1 RECEPTOR

DEUTERIERTE (TRIFLUORMETHYL)PYRIMIDIN-2-AMIN-VERBINDUNGEN ALS VERSTÄRKER DES HMRGX1-REZEPTORS

COMPOSÉS DE (TRIFLUOROMÉTHYL)PYRIMIDIN-2-AMINE DEUTÉRÉS UTILISÉS EN TANT QUE POTENTIALISATEURS DU RÉCEPTEUR HMRGX1

(84) Designated Contracting States:
**AL AT BE BG CH CY CZ DE DK EE ES FI FR GB GR HR HU IE IS IT LI LT LU LV MC ME MK MT NL NO PL PT RO RS SE SI SK SM TR**

(30) Priority: **08.11.2021 US 202163276748 P**

(43) Date of publication of application:
**18.09.2024 Bulletin 2024/38**

(73) Proprietor: **ELI LILLY AND COMPANY**
**Indianapolis, IN 46285 (US)**

(72) Inventors:
- **RUBLE, James Craig**
  **Indianapolis, IN 46206-6288 (US)**
- **WINNEROSKI, Leonard Larry**
  **Indianapolis, IN 46206-6288 (US)**

(74) Representative: **J A Kemp LLP**
**80 Turnmill Street**
**London EC1M 5QU (GB)**

(56) References cited:
**WO-A1-2021/225878** **WO-A2-2008/052072**

- **FOSTER A B: "Deuterium isotope effects in the metabolism of drugs and xenobiotics: implications for drug design", ADVANCES IN DRUG RESEARCH, ACADEMIC PRESS, LONDON, GB, vol. 14, 1 January 1985 (1985-01-01), pages 1 - 40, XP009086953, ISSN: 0065-2490**

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

EP 4 429 764 B1

## Description

The present invention relates to compounds that are potentiators of the hMrgX1 receptor, to pharmaceutical compositions comprising the compounds, to the compounds to treat pain, and to intermediates and processes useful in the synthesis of the compounds.

It is estimated that about 20% of adults in the United States suffer from chronic pain. Chronic pain is one of the most common reasons adults seek medical care and is linked to restrictions in mobility and daily activities. Unfortunately, chronic pain is often refractory to current therapies and many analgesics are associated with dose-limiting adverse events or serious risk of addiction and abuse which can be substantial barriers to their use in treating chronic pain.

United States Patent No. 6,326,368 discloses certain 2-aryloxy- and 2-arylthiosubstituted pyrimidines and triazines and derivatives thereof as corticotropin releasing factor (CRF) receptor antagonists useful in treating various disorders, such as depression, anxiety, drug addiction, and inflammatory disorders. United States Patent No. 5,100,459 discloses certain substituted sulfonylureas and intermediates thereof. W. Wangdong, et. al., ChemMedChem, vol 10(1), 57-61 (2015) discloses 2-(cyclopropanesulfonamido)-N-(2-ethoxyphenyl)benzamide, ML382, as a potent and selective positive allosteric modulator of MrgX1. United States Patent No. 11,414,389 discloses (trifluoromethyl)pyrimidine-2-amine compounds as potentiators of human MrgX1. WO2008/052072 discloses compounds useful for the treatment of pain.

There is a need for alternative treatments of pain, including chronic pain. In addition, there is a need for compounds that are potentiators of the hMrgX1 receptor. Furthermore, hMrgX1 receptor potentiators possessing certain properties that make them suitable for administration to humans, including favorable pharmacokinetic properties, such as favorable metabolic properties, are desired. In addition, hMrgX1 receptor potentiators that are CNS penetrant are desired. The present invention provides compounds that address one or more of these needs.

Accordingly, in one embodiment, the present invention provides a compound of Formula I:

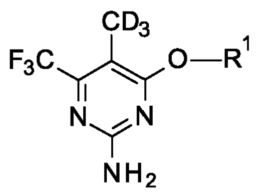

Formula I

wherein $R^1$ is:

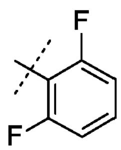

or

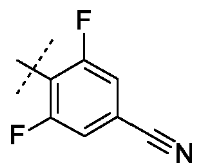

or a pharmaceutically acceptable salt thereof.

A particular embodiment is the compound of Formula Ia:

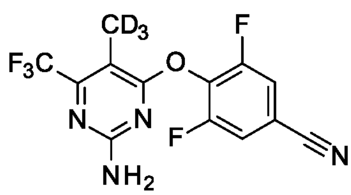

Formula Ia

or a pharmaceutically acceptable salt thereof.

In addition, a particular embodiment is the compound of Formula Ia:

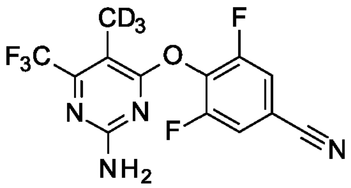

Formula Ia

A particular embodiment is the compound of Formula Ib:

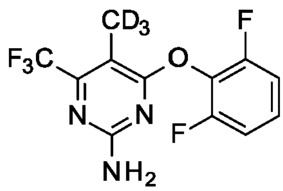

Formula Ib

or a pharmaceutically acceptable salt thereof.

In addition, a particular embodiment is the compound of Formula Ib:

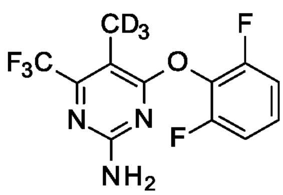

Formula Ib

.

In a particular embodiment, each position represented as D in Formula I, Ia, or Ib has deuterium enrichment of at least 20%, or a pharmaceutically acceptable salt thereof.

In a particular embodiment, each position represented as D in Formula I, Ia, or Ib has deuterium enrichment of at least 30%, or a pharmaceutically acceptable salt thereof.

In a particular embodiment, each position represented as D in Formula I, Ia, or Ib has deuterium enrichment of at least 40%, or a pharmaceutically acceptable salt thereof.

In a particular embodiment, each position represented as D in Formula I, Ia, or Ib has deuterium enrichment of at least 50%, or a pharmaceutically acceptable salt thereof.

In a particular embodiment, each position represented as D in Formula I, Ia, or Ib has deuterium enrichment of at least 60%, or a pharmaceutically acceptable salt thereof.

In a particular embodiment, each position represented as D in Formula I, Ia, or Ib has deuterium enrichment of at least 70%, or a pharmaceutically acceptable salt thereof.

In a particular embodiment, each position represented as D in Formula I, Ia, or Ib has deuterium enrichment of at least 80%, or a pharmaceutically acceptable salt thereof.

In a particular embodiment, each position represented as D in Formula I, Ia, or Ib has deuterium enrichment of at least 85%, or a pharmaceutically acceptable salt thereof.

In a particular embodiment, each position represented as D in Formula I, Ia, or Ib has deuterium enrichment of at least 90%, or a pharmaceutically acceptable salt thereof.

In a particular embodiment, each position represented as D in Formula I, Ia, or Ib has deuterium enrichment of at least 93%, or a pharmaceutically acceptable salt thereof.

In a particular embodiment, each position represented as D in Formula I, Ia, or Ib has deuterium enrichment of at least 95%, or a pharmaceutically acceptable salt thereof.

In a particular embodiment, each position represented as D in Formula I, Ia, or Ib has deuterium enrichment of at least 99%, or a pharmaceutically acceptable salt thereof.

In a particular embodiment, each position represented as D in Formula I, Ia, or Ib has deuterium enrichment of at least 20%.

In a particular embodiment, each position represented as D in Formula I, Ia, or Ib has deuterium enrichment of at least 30%.

In a particular embodiment, each position represented as D in Formula I, Ia, or Ib has deuterium enrichment of at least 40%.

In a particular embodiment, each position represented as D in Formula I, Ia, or Ib has deuterium enrichment of at

least 50%.

In a particular embodiment, each position represented as D in Formula I, Ia, or Ib has deuterium enrichment of at least 60%.

In a particular embodiment, each position represented as D in Formula I, Ia, or Ib has deuterium enrichment of at least 70%.

In a particular embodiment, each position represented as D in Formula I, Ia, or Ib has deuterium enrichment of at least 80%.

In a particular embodiment, each position represented as D in Formula I, Ia, or Ib has deuterium enrichment of at least 85%.

In a particular embodiment, each position represented as D in Formula I, Ia, or Ib has deuterium enrichment of at least 90%.

In a particular embodiment, each position represented as D in Formula I, Ia, or Ib has deuterium enrichment of at least 93%.

In a particular embodiment, each position represented as D in Formula I, Ia, or Ib has deuterium enrichment of at least 95%.

In a particular embodiment, each position represented as D in Formula I, Ia, or Ib has deuterium enrichment of at least 99%.

In an embodiment, the present invention also provides the compounds of Formula I, Ia or Ib for use in a method of treating pain in a patient in need of such treatment, comprising administering to the patient an effective amount of a compound of Formula I, Ia, or Ib, or a pharmaceutically acceptable salt thereof. In an embodiment, the present invention further provides the compounds of Formula I, Ia or Ib for use in a method of treating chronic pain in a patient in need of such treatment, comprising administering to the patient an effective amount of a compound of Formula I, Ia, or Ib, or a pharmaceutically acceptable salt thereof. In an embodiment, the present invention further provides the compounds of Formula I, Ia or Ib for use in a method of treating chronic lower back pain in a patient in need of such treatment, comprising administering to the patient an effective amount of a compound of Formula I, Ia, or Ib, or a pharmaceutically acceptable salt thereof. In an embodiment, the present invention further provides the compounds of Formula I, Ia or Ib for use in a method of treating diabetic peripheral neuropathic pain in a patient in need of such treatment, comprising administering to the patient an effective amount of a compound of Formula I, Ia, or Ib, or a pharmaceutically acceptable salt thereof. In an embodiment, the present invention further provides the compounds of Formula I, Ia or Ib for use in a method of treating osteoarthritis pain in a patient in need of such treatment, comprising administering to the patient an effective amount of a compound of Formula I, Ia, or Ib, or a pharmaceutically acceptable salt thereof.

In an embodiment, the present invention further provides a compound of Formula I, Ia, or Ib, or a pharmaceutically acceptable salt thereof for use in therapy. In an embodiment, the present invention provides a compound of Formula I, Ia, or Ib, or a pharmaceutically acceptable salt thereof for use in treating pain. In an embodiment, the present invention provides a compound of Formula I, Ia, or Ib, or a pharmaceutically acceptable salt thereof, for use in treating chronic pain. In an embodiment, the present invention provides a compound of Formula I, Ia, or Ib, or a pharmaceutically acceptable salt thereof, for use in treating chronic lower back pain. In an embodiment, the present invention provides a compound of Formula I, Ia, or Ib, or a pharmaceutically acceptable salt thereof, for use in treating diabetic peripheral neuropathic pain. In an embodiment, the present invention provides a compound of Formula I, Ia, or Ib, or a pharmaceutically acceptable salt thereof, for use in treating osteoarthritis pain.

In an embodiment, the present invention also provides the use of a compound of Formula I, Ia, or Ib, or a pharmaceutically acceptable salt thereof, for the manufacture of a medicament for treating pain. In an embodiment, the present invention provides the use of a compound of Formula I, Ia, or Ib, or a pharmaceutically acceptable salt thereof, for the manufacture of a medicament for treating chronic pain. In an embodiment, the present invention provides the use of a compound of Formula I, Ia, or Ib, or a pharmaceutically acceptable salt thereof, for the manufacture of a medicament for treating chronic lower back pain. In an embodiment, the present invention provides the use of a compound of Formula I, Ia, or Ib, or a pharmaceutically acceptable salt thereof, for the manufacture of a medicament for treating diabetic peripheral neuropathic pain. In an embodiment, the present invention provides the use of a compound of Formula I, Ia, or Ib, or a pharmaceutically acceptable salt thereof, for the manufacture of a medicament for treating osteoarthritis pain.

In an embodiment, the present invention further provides a pharmaceutical composition, comprising a compound of Formula I, Ia, or Ib, or a pharmaceutically acceptable salt thereof, with one or more pharmaceutically acceptable carriers, diluents, or excipients. In an embodiment, the present invention further provides a pharmaceutical composition, comprising a compound of Formula I, Ia, or Ib with one or more pharmaceutically acceptable carriers, diluents, or excipients. In an embodiment, the present invention further provides a process for preparing a pharmaceutical composition, comprising admixing a compound of Formula I, Ia, or Ib, or a pharmaceutically acceptable salt thereof, with one or more pharmaceutically acceptable carriers, diluents, or excipients. In an embodiment, the present invention further provides a process for preparing a pharmaceutical composition, comprising admixing a compound of Formula I, Ia, or Ib with one or more pharmaceutically acceptable carriers, diluents, or excipients. In an embodiment, the present invention

also encompasses novel intermediates and processes for the synthesis of compounds of Formula I, Ia, and Ib.

As used herein, the terms "treating", "treatment", or "to treat" includes restraining, slowing, stopping, or reversing the progression or severity of an existing symptom or disorder.

As used herein, the term "patient" refers to a mammal, in particular a human.

As used herein, the term "effective amount" refers to the amount or dose of compound of the invention, or a pharmaceutically acceptable salt thereof which, upon single or multiple dose administration to the patient, provides the desired effect in the patient under diagnosis or treatment.

An effective amount can be determined by one skilled in the art by the use of known techniques and by observing results obtained under analogous circumstances. In determining the effective amount for a patient, a number of factors are considered by the attending diagnostician, including, but not limited to: the species of patient; its size, age, and general health; the specific disease or disorder involved; the degree of or involvement or the severity of the disease or disorder; the response of the individual patient; the particular compound administered; the mode of administration; the bioavailability characteristics of the preparation administered; the dose regimen selected; the use of concomitant medication; and other relevant circumstances.

As used herein the symbol "D" refers to a deuterium atom.

As used herein "deuterium enrichment" refers to the percentage of incorporation of a deuterium atom at a given position in the compound of Formula I, Ia, or Ib in the place of a hydrogen atom. For example, deuterium enrichment of at least 90% at a specified position means that 90% or more of the molecules in a sample contain deuterium at the specified position. The deuterium enrichment of a sample can be determined using standard analytical techniques and methods well known to one of ordinary skill in the art, including but not limited to, mass spectrometry and nuclear magnetic resonance spectroscopy. In addition, unless otherwise stated, when a position is specifically designated as "D" or "deuterium", the position is understood to have deuterium enrichment of at least 20%.

As used herein, "$C_{1-4}$ alkyl" refers to an alkyl substituent having from 1 to 4 carbon atoms which can be branched or unbranched, and include for example, methyl, ethyl, propyl, isopropyl, butyl, and the like, with methyl and ethyl being preferred.

As used herein, "aryl" refers to a carbocyclic aromatic substituent containing 6 carbon atoms which may be unsubstituted or substituted, including phenyl, 4-methylphenyl, and the like.

As used herein, "tosylate" refers to a p-toluene sulfonate substituent.

The compounds of the present invention are formulated as pharmaceutical compositions administered by any route which makes the compound bioavailable. Most preferably, such compositions are for oral administration. Such pharmaceutical compositions and processes for preparing same are well known in the art (See, e.g., Remington: The Science and Practice of Pharmacy, A. Adejare, Editor, 23nd Edition, published 2020, Elsevier Science).

A pharmaceutically acceptable salt of a compound of the invention can be formed, for example, by reaction of an appropriate free base of a compound of the invention, an appropriate pharmaceutically acceptable acid in a suitable solvent such as diethyl ether under standard conditions well known in the art. See, for example, Gould, P.L., "Salt selection for basic drugs," International Journal of Pharmaceutics, 33: 201-217 (1986); Bastin, R.J., et al. "Salt Selection and Optimization Procedures for Pharmaceutical New Chemical Entities," Organic Process Research and Development, 4: 427-435 (2000); and Berge, S.M., et al., "Pharmaceutical Salts," Journal of Pharmaceutical Sciences, 66: 1-19, (1977).

Certain abbreviations are defined as follows: "ACN" refers to acetonitrile; "MTBE" refers to methyl tert-butyl ether; "THF" refers to tetrahydrofuran; "DMF" refers to dimethylformamide; "EtOAc" refers to ethyl acetate: "BAM8-22" refers to bovine adrenal medulla peptide 8-22; "Cat. #" refers to catalog number; "CRC" refers to concentration-response curve; "DMEM" refers to Dulbecco's modified eagle media; "DMSO" refers to dimethyl sulfoxide; "DPBS" refers to Dulbecco's phosphate-buffered saline; "$EC_{50}$" refers to the effective concentration of an agent that gives a half-maximal response between baseline and maximum after a specified exposure time; "EDTA" refers to ethylenediaminetetraacetic acid; "ESMS" refers to Electrospray Mass Spectrometry; "FBS" refers to fetal bovine serum; "g" refers to gram or grams; "h" refers to hour or hours; "HEC" refers to hydroxyethylcellulose; "HEK293" refers to human embryonic kidney 293 cell or cells; "HEPES" refers to (4-(2-hydroxyethyl)-1-piperazineethanesulfonic acid); "hMrgX1" refers to human MrgX1 receptor; "HTRF" refers to homogeneous time resolved fluorescence; "IP1" refers to inositol monophosphate; "$K_{p,uu}$" refers to unbound brain-to-plasma partition coefficient; "LC-ESMS" refers to refers to Liquid Chromatography Electrospray Mass Spectrometry; "min" refers to minute or minutes; "mL" refers to milliliter or milliliters; "mol" refers to mole or moles; "mmol" refers to millimole or millimoles; "nm" refers to nanometer or nanometers; "nmol" refers to nanomoles; "m/z" refers to mass-to-charge ration for mass spectroscopy; "n," when in the context of biological data, refers to the number of runs or number of times tested; "PBS" refers to phosphate-buffered saline; "rpm" refers to revolutions per minute or minutes; "SD" refers to standard deviation; "SEM" refers to standard error of the mean; "U/mL" refers to units per milliliter; "V," when in the context of solvents, refers to volumes.

The compounds of the present invention, or salts thereof, may be prepared by a variety of procedures known to one of ordinary skill in the art, some of which are illustrated in the schemes, preparations, and examples below. One of ordinary skill in the art recognizes that the specific synthetic steps for each of the routes described may be combined in

different ways, or in conjunction with steps from different schemes, to prepare compounds of the invention, or salts thereof. The products of each step in the schemes below can be recovered by conventional methods well known in the art, including extraction, evaporation, precipitation, chromatography, filtration, trituration, and crystallization. In the schemes below, all substituents unless otherwise indicated, are as previously defined. The reagents and starting materials are readily available to one of ordinary skill in the art. The following schemes, preparations, examples, and assays further illustrate the invention, but should not be construed to limit the scope of the invention in any way.

Scheme 1

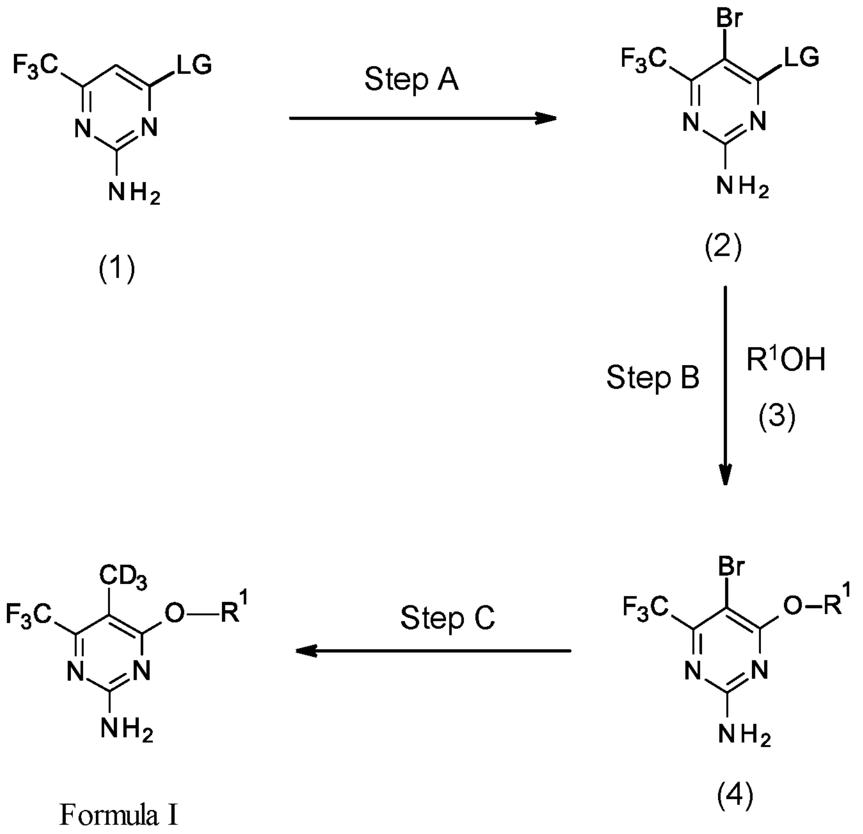


(1) (2) (4) Formula I

$R^1OH$ is:

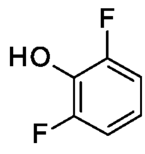
or
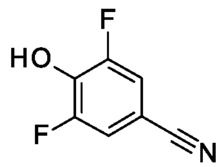

In Scheme 1, step A, a compound of structure (1), wherein LG is a suitable leaving group, such as F, Cl, Br, I, tosylate, or $-SO_2R^a$ wherein $R^a$ is aryl or alkyl, such as phenyl or methyl (with Cl being preferred, such as 4-chloro-6-(trifluoromethyl)pyrimidin-2-amine), is dissolved in a suitable organic solvent, such as ACN with stirring under an inert atmosphere, such as nitrogen, and then treated with about 1.1 equivalents of 3-dibromo-5,5-dimethyl-imidazolidine-2,4-dione. The reaction is allowed to stir for about 12 to 24 hours. The product is then isolated using techniques well known in the art such as dilution with water, extraction with a suitable organic solvent, such as EtOAc, drying the combined organic extracts over sodium sulfate, filtering, and concentrating under reduced pressure to provide the compound of structure (2).

In Scheme 1, step B, the compound of structure (2) is combined with about 1.1 equivalents of the compound of structure (3) in a suitable organic solvent, such as ACN. The mixture is then treated with about 1.5 equivalents of a suitable base, such as cesium carbonate or potassium carbonate, and the reaction mixture is heated at reflux for about 3 hours. The reaction is then cooled to room temperature and the product isolated using standard techniques well known in the art, such as dilution with water, extraction with a suitable organic solvent, such as EtOAc or MTBE, drying the combined organic extracts over sodium sulfate, filtering, and concentrating under reduced pressure to provide the compound of structure (4).

This material can be purified using flash chromatography on silica gel with a suitable eluent, such as EtOAc:hexanes to provide the purified compound of structure (4).

In Scheme 1, step C, the compound of structure (4) is combined with about 1.25 equivalents of 5-(trideuteriomethyl)-1-aza-5-stannabicyclo[3.3.3]undecane and about 0.05 equivalents of a suitable catalyst, such as bis(tri-tert-butylphosphine)palladium(0) under an inert atmosphere, such as nitrogen, in an oven-dried Biotage 10-20 mL microwave vial containing a stir bar. A suitable organic solvent, such as DMF is added, the vial is capped and heated with stirring at about 100 °C for about 1-2 hours. The reaction mixture is then combined with a suitable solvent mixture, such as MTBE and 1 M aqueous KF. The organics are then separated and washed successively with 1 M aqueous KF, water, and saturated aqueous NaCl. The organics were then dried over $MgSO_4$, filtered, and the filtrate concentrated under reduced pressure to provide the crude compound of Formula I. This crude material can then be purified using standard techniques well known in the art, such as chromatography on silica gel with a suitable eluent, such as EtOAc:hexane to provide the purified compound of Formula I which includes within its scope, Formula Ia and Formula Ib. One of ordinary skill in the art recognizes that the deuterium enrichment of the reagent used in this step C, 5-(trideuteriomethyl)-1-aza-5-stannabicyclo[3.3.3]undecane, will determine the deuterium enrichment of the final compound of Formula I, Ia, and Ib.

Preparation 1

5-(trideuteriomethyl)-1-aza-5-stannabicyclo[3.3.3]undecane

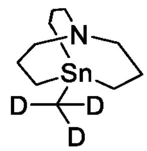

5-Chloro-1-aza-5-stannabicyclo[3.3.3]undecane (2.03 g, 6.90 mmol) and THF (50 mL) were added under nitrogen to an oven-dried flask containing a stir bar. The resulting slurry was cooled in a dry ice/ACN bath, and methyl-d3-magnesium iodide (1 M in diethyl ether, 14 mL, 14 mmol, >99% deuterium enriched) was added over 10 minutes. The mixture was stirred in the dry ice/ACN bath for 2.5 hours and then in an ice bath for 20 minutes. 35 minutes after the ice bath was removed, the mixture was poured into a separatory funnel with water (100 mL) and MTBE (100 mL). The organic layer was further washed with brine (100 mL), dried over $MgSO_4$, filtered, and evaporated. The residue was dried under vacuum yielding the title compound as 1.71 g of white solid. $^1H$ and $^{13}C$ NMR data were generally consistent with those reported in Angew. Chem. Int. Ed. 2015, 54, 5488-5492.

Preparation 2

5-bromo-4-chloro-6-(trifluoromethyl)pyrimidin-2-amine

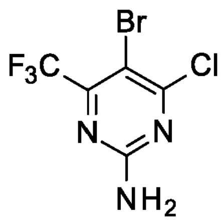

Scheme 1, step A: A room temperature solution of 4-chloro-6-(trifluoromethyl)pyrimidin-2-amine (20 g, 98.2 mmol) in ACN (200 mL) was treated portion-wise with 1,3-dibromo-5,5-dimethyl-imidazolidine-2,4-dione (31.52 g, 108 mmol) and the reaction was stirred overnight under nitrogen. An orange slurry had formed that was diluted with water and extracted three times with ethyl acetate. Combined the organic extracts and dried over sodium sulfate. Filtered and evaporated the resulting filtrate under reduced pressure to afford a crude solid that was triturated in hexanes/ethyl acetate and filtered to afford the title compound (13.82 g, 54% yield). ESMS (m/z, $^{79}Br/^{81}Br$): 370/372 [M+H].

Preparation 3

5-bromo-4-(2,6-difluorophenoxy)-6-(trifluoromethyl)pyrimidin-2-amine

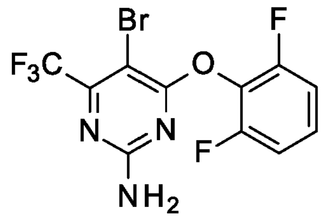

Scheme 1, step B: Combined 5-bromo-4-chloro-6-(trifluoromethyl)pyrimidin-2-amine (19.46 g, 69 mmol) and 2,6-difluorophenol (9.87 g, 75.9 mmol) in ACN (200 mL). Added cesium carbonate (33.72 g, 103.5 mmol) and heated to reflux for 3 hours. Cooled the reaction mixture, diluted with water, and extracted three times with ethyl acetate. Combined the organic extracts and dried over sodium sulfate. Filtered and evaporated the resulting filtrate under reduced pressure to afford a crude solid that was purified by flash chromatography over silica gel, eluting with 5-100% ethyl acetate in hexanes, to afford the title compound (16.57 g, 61% yield), after solvent evaporation of the desired chromatographic fractions. ESMS (m/z, $^{79}Br/^{81}Br$): 274/276 [M-H].

Preparation 4

4-((2-amino-5-bromo-6-(trifluoromethyl)pyrimidin-4-yl)oxy)-3,5-difluorobenzonitrile

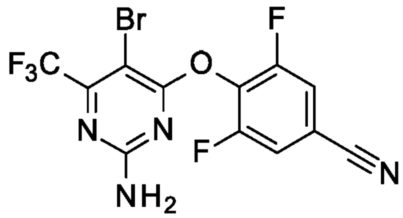

Scheme 1, step B: 5-Bromo-4-chloro-6-(trifluoromethyl)pyrimidin-2-amine (2.01 g, 7.27 mmol), 3,5-difluoro-4-hydroxy-benzonitrile (2.25 g, 14.5 mmol), potassium carbonate (2.02 g, 14.6 mmol), and DMF (20 mL) were charged to a flask with a stir bar and heated on an 80 °C stir block for 3 hours. The mixture was cooled to room temperature and then added to a separatory funnel with water and MTBE. The organic layer was then washed with 2 additional portions of water followed by saturated aqueous sodium chloride. The organics were dried over $Na_2SO_4$ and evaporated. The product was then purified by flash chromatography on silica gel using 0% to 30% ethyl acetate in hexane. After concentration of the product fractions, the residue was re-purified by flash chromatography on silica gel using 0% to 30% MTBE in hexane. Evaporation of the desired fractions yielded the title compound as 2.50 g of white solid. ESMS (m/z, $^{79}Br/^{81}Br$): 395/397 [M+H].

Example 1

4-[2-amino-5-(trideuteriomethyl)-6-(trifluoromethyl)pyrimidin-4-yl]oxy-3,5-difluoro-benzonitrile

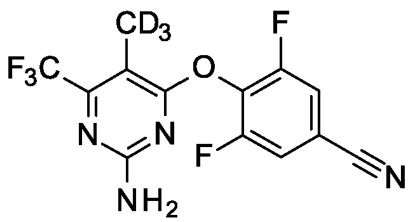

Scheme 1, step C: 4-((2-Amino-5-bromo-6-(trifluoromethyl)pyrimidin-4-yl)oxy)-3,5-difluorobenzonitrile (600 mg, 1.52 mmol), 5-(trideuteriomethyl)-1-aza-5-stannabicyclo[3.3.3]undecane (530 mg, 1.91 mmol; deuterium enrichment of at least 95% at each deuterium) and bis(tri-tert-butylphosphine)palladium(0) (45 mg, 0.088 mmol) were added under nitrogen as solids to an oven-dried Biotage 10-20 mL microwave vial containing a stir bar. DMF (9.0 mL) was added, and the vial was capped. The vial was heated with stirring in a 100 °C block for 105 minutes. The mixture was then added to a separatory funnel with MTBE (100 mL) and 1 M aqueous KF (100 mL). The organics were separated and then washed successively with 1 M aqueous KF (100 mL), water (100 mL) and brine (100 mL). The organics were dried over $MgSO_4$ and filtered through a 1 inch silica plug. The plug was rinsed with MTBE, and the combined filtrates were evaporated. The residue was dissolved in DCM and adsorbed onto silica gel. The product was then purified by chromatography on silica gel using 20% to 30% ethyl acetate in hexane. Evaporation of the desired fractions followed by drying at 60 °C under vacuum yielded the title compound as 327 mg of white solid; deuterium enrichment of at least 95% at each deuterium; ESMS (m/z): 334 [M+H].

Following purification, one batch of the compound of Example 1 was subjected to proton NMR in order to quantify incorporation of deuterium. Signal from the methyl group integrated to <0.005, which, compared to the 3.00 value which would be obtained in a protonated group, indicated greater than 99% incorporation of deuterium.

Example 2

4-(2,6-difluorophenoxy)-5-(trideuteriomethyl)-6-(trifluoromethyl)pyrimidin-2-amine

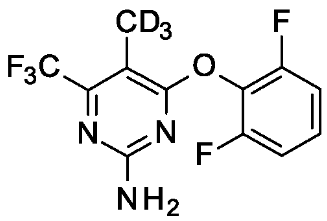

Scheme 1, step C: 5-Bromo-4-(2,6-difluorophenoxy)-6-(trifluoromethyl)pyrimidin-2-amine (576 mg, 1.56 mmol), 5-(trideuteriomethyl)-1-aza-5-stannabicyclo[3.3.3]undecane (535 mg, 1.93 mmol; deuterium enrichment of at least 95% at each deuterium), and bis(tri-tert-butylphosphine)palladium(0) (57 mg, 0.11 mmol) were added under nitrogen as solids to an oven-dried Biotage 10-20 mL microwave vial containing a stir bar. DMF (9.0 mL) was added, and the tube was capped. The vial was heated with stirring in a 100 °C block for 3 hours. The mixture was then added to a separatory funnel with MTBE (100 mL) and 1 M aqueous KF (100 mL). The organics were separated and then washed successively with 1 M aqueous KF (100 mL), water (100 mL) and brine (100 mL). The organics were filtered through a 1" silica plug. The plug was rinsed with MTBE, and the combined filtrates were evaporated. The residue was dissolved in DCM and adsorbed onto silica gel. The product was then purified by chromatography on silica gel using 20% to 25% ethyl acetate in hexane. Evaporation of the desired fractions followed by drying at 60 °C under vacuum yielded the title compound as 376 mg of white solid; deuterium enrichment of at least 95% at each deuterium; ESMS (m/z): 309 [M+H].

Scheme 2

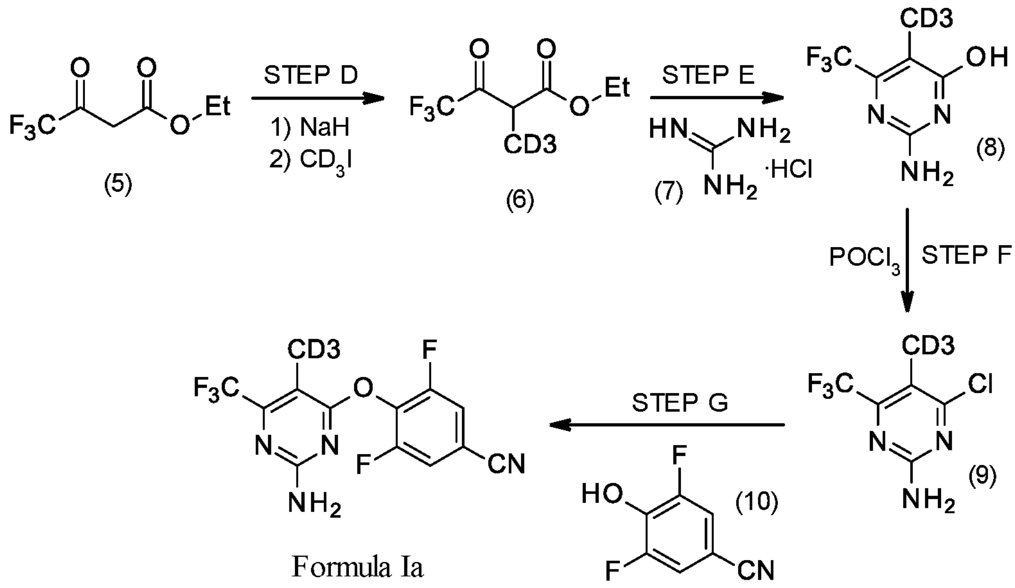


Preparation 5

ethyl 4,4,4-trifluoro-3-oxo-2-(trideuteriomethyl)butanoate

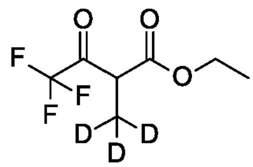

Scheme 2, Step D: A mixture of 1,2-dimethoxyethane (2500 mL) and sodium hydride (24 g, 600.1 mmol) was cooled to 0 °C and treated dropwise with a solution of ethyl 4,4,4-trifluoro-3-oxo-butanoate (100 g, 543.15 mmol) in 1,2-dimethoxyethane (300 mL) over 20 min. The mixture was stirred for 30 min at 25 °C and then trideuterio(iodo)methane (158 g, 1090 mmol) was added at 25 °C and the reaction was heated to 90 °C and stirred for 3 h. The reaction was cooled and quenched with aq $NH_4Cl$ (200 mL) and extracted with MTBE (200 mL × 2). The combined organic layers were washed with brine (100 mL) and dried over $Na_2SO_4$. The combined organic layers were filtered by filter paper to give the filtrate which was concentrated under vacuum at 35 °C to afford 100 g (91%) of the title product as a yellow oil. $^{19}F$ (DMSO-d6) = -81.85 ppm

Preparation 6

2-amino-5-(trideuteriomethyl)-6-(trifluoromethyl) pyrimidin-4-ol

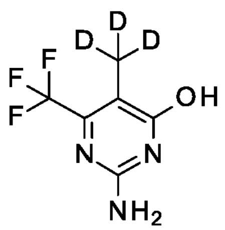

Scheme 2, Step E: A 0 °C solution of ethyl 4,4,4-trifluoro-3-oxo-2-(trideuteriomethyl)butanoate (60 g, 298.3 mmol) in methanol (600 mL) was treated with guanidine hydrochloride (30 g, 314.0 mmol) followed by a solution of sodium methoxide in methanol (100 mL, 400 mmol) at 25 °C. The reaction was heated to 90 °C and stirred for 3 h. The reaction mixture was cooled and concentrated in vacuum to remove methanol. The crude was acidified with acetic acid (27.5 mL, 480 mmol) in water (600 mL) and stirred at 25 °C for 10 minutes to afford a white solid precipitated that was filtered and washed with water (100 mL × 2) to give the filter cake that was dried under vacuum at 40 °C to afford 41 g (64%) of the title product. ESMS (m/z): 197 [M+H]

Preparation 7

4-chloro-5-(trideuteriomethyl)-6-(trifluoromethyl) pyrimidin-2-amine

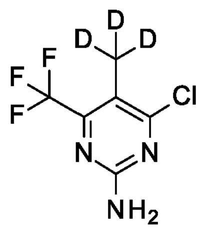

Scheme 2, Step F: A 0 °C mixture of 2-amino-5-(trideuteriomethyl)-6-(trifluoromethyl)pyrimidin-4-ol (16 g, 81.57 mmol) in acetonitrile (160 mL) was treated with triethylamine (11.4 mL, 81.8 mmol) and then treated dropwise with phosphoryl chloride (8.4 mL, 90 mmol). The reaction was heated to 80 °C and stirred for 12 h. The reaction mixture was concentrated in vacuum to remove phosphoryl chloride to a volume of ~100 mL and added dropwise into water (500 mL) and stirred at 25 °C for 0.5 h to afford a solid that was filtered, and the filter cake was dried under vacuum at 40 °C to afford 8 g (45%) of the title product. ESMS (m/z, $^{35}Cl/^{37}Cl$): 215/217 [M+H]

Example 1 (Alternate Preparation)

4-[2-amino-5-(trideuteriomethyl)-6-(trifluoromethyl)pyrimidin-4-yl]oxy-3,5-difluoro-benzonitrile

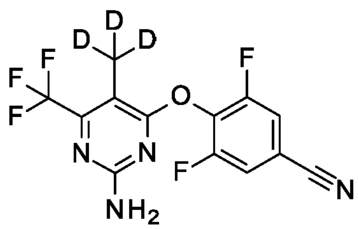

Scheme 2, Step G: A solution of 4-chloro-5-(trideuteriomethyl)-6-(trifluoromethyl)pyrimidin-2-amine (37 g, 172.4 mmol,) and 3,5-difluoro-4-hydroxybenzonitrile (54 g, 348.15 mmol) in N,N-dimethylacetamide (370 mL) was treated with potassium phosphate tribasic (187 g, 863.3 mmol) and heated to 100 °C for 12 h. The reaction mixture was added dropwise over 10 min into water (50 V) to afford a white solid precipitate that was filtered. The filter cake was dried under vacuum to afford crude product that was triturated in acetonitrile (2 V) and filtered to afford 35 g of solid. The solid was recrystallized with EA (3 V) and heptane (10 V) and filtered, and the filter cake was dried under vacuum to afford 30 g solid that was recrystallized again with EA (3 V)/Heptane(10 V) to afford 26 g (45%) of the title product. ESMS (m/z): 344 [M+H]

**IP1 Cellular Assay for $EC_{50}$ Determination against hMrgX1 by HTRF**

*Cell plating:* HEK293 cells stably expressing the recombinant human MrgX1 receptor were expanded in culture flasks (Corning, T150), using growth media containing DMEM with glutamine (GIBCO™, Cat. # 11960-044) supplemented with 10% heat-inactivated FBS (HyCloneTM, Cat. # CH30073), 1% penicillin/streptomycin (HyClone™, Cat. # SV30010; 10,000 U/mL penicillin; 10,000 μg/mL streptomycin in 0.85% NaCl), 20mM HEPES (GIBCO™, Cat. # 15630122) and 0.3

mg/mL G418 (GIBCO™, Cat. # 11811031). When cell monolayers achieved a level of 80-90% confluence, monolayers were washed once with 10 mL of DPBS (HyClone™, Cat. # 14190-144), dissociated using TrypLE™ Express enzyme cell dissociation media (GIBCO™, Cat. # 12605-010), and were diluted by addition of 10 mL DPBS. Dissociated cells were transferred to a sterile 50 mL conical tube, pelleted by centrifugation at 300 x *g* to remove the growth and dissociation media, and diluted to 1M cells/mL into DMEM for plating.

*IP1 Potency and Efficacy Determination:* Test compounds were dissolved in DMSO to a concentration of 10 mM and serially diluted in DMSO to obtain a 10-point concentration response stock dilution plate. Growth media was removed from the cell plate, and the stock 10-point dilution plate was diluted into media and stamped into the cell plate at a concentration 2x higher than the final test concentration of 30 μM maximum. The endogenous agonist BAM8-22 (Tocris-BioScience® Cat. # 1763) was diluted to the $EC_{15}$, determined independently at a minimum of n = 3, into the cell plate and incubated at room temperature for 120 min. Subsequently, half the volume each of anti-IP1 cryptate and d2-labeled IP1 in lysis buffer, supplied with the IP-One Gq Kit (CisBio Cat. # 62IPAPEC) was added to the cell plate to initiate cell lysis, and incubated for 60 min at room temperature in the dark. Fluorescence was then determined at 620 and 665 nm (~100 μs following laser excitation).

*Data Analysis:* Fluorescent ratios were determined as the ratio of the fluorescence emission at 665 nm over 620 nm and converted to IP1 concentration, using the IP1 standard curve generated in a separate plate, following the manufacturer's instructions. The IP1 concentration was then plotted as a function of compound concentration. Potentiator potency ($EC_{50}$) is defined as the compound concentration, in the presence of the $EC_{15}$ of the endogenous agonist BAM8-22, resulting in 50% of the increase in IP1 concentration achieved by a saturating concentration of BAM8-22, and was determined by using Genedata software (GeneData AG, Basel Switzerland) fitting the following equation to the 10-point CRC, where y is the IP1 concentration determined for a given compound concentration, [L] denotes the concentration of test compound and Max is the maximum increase achieved by a saturating concentration of BAM8-22:

$$Y=Max*[L]/(EC_{50} + [L])$$

$EC_{50}$ values are reported as the geometric mean in nM (SEM, n).

**Table 1. Relative $EC_{50}$ against hMrgX1 IP-1 for the compounds of Examples 1 and 2**

| Example | Relative $EC_{50}$ (SEM, n) (nM) | Max (Mean ± SEM) (%) |
|---|---|---|
| 1 | 22 (5, 4) | 111 ± 14.9, n=4 |
| 2 | 28 (11, 4) | 106 ± 8.16, n=4 |

Table 1 shows the relative $EC_{50}$ and the maximum stimulation achieved in the assay, as essentially described above, for the compounds of Examples 1 and 2, indicating these compounds are potentiators of hMrgX1.

**In vivo determination of $K_{p,uu,brain}$ in mice**

Unbound brain-to-plasma partition coefficient ($K_{p,uu,brain}$) is one of the key pharmacokinetic parameters for evaluating a compound's ability to cross the blood-brain barrier (BBB). It is typically measured in pre-clinical species using the following methodology. $K_{p,uu,brain}$ values indicate the fraction of free drug in plasma that partitions across the BBB.

*Subjects:* The subjects for these studies were 12 male CD1 (ICR) mice (Envigo, Indianapolis, IN, USA) between 5-7 weeks old at time of test. Mice were housed in groups of 4 in high density plastic home cages. Food and water were available ad libitum. The rooms were maintained at 73 °F with 30-70% relative humidity and kept on a light/dark cycle of 0600-1800 h.

*Agent:* The compound of Example 1 was prepared at 10 mg/ml in the 1% HEC, 0.25% TWEEN®80, 0.05% DOWSIL™ vehicle in water. Prepared compound was sonicated in water bath for 30 min until a suspension was formed. Mice were dosed orally at 10 ml/kg for a 100 mg/kg dose.

*Dosing and Tissue Collection:* For this experiment, ten mice received oral dosing of 100 mg/kg of the compound of Example 1. Mice were then euthanized at 2 h post-dosing via $CO_2$ asphyxiation, plasma samples were collected via cardia puncture, and mouse brains removed, weighed, and frozen on dry ice. Blood samples were stored in EDTA tubes on wet ice and centrifuged at 15k rpm for 10 min. Plasma was collected, plated in a 96-well plate, and frozen at -80 °C.

*Pharmacokinetic sampling:* Plasma and brain samples obtained were analyzed for Example 1 using an LC-MS/MS method (Q2 Solutions, Indianapolis, IN, USA). Plasma samples were extracted using protein precipitation. The lower limit of quantification was 25 ng/mL, and the upper limit of quantification was 5000 ng/mL. Brain samples were homogenized, and the analyte was extracted using protein precipitation. The lower limit of quantification was 4 ng/g and the upper limit of quantification was 200000 ng/g.

*Determination of plasma and brain protein binding:* Mouse plasma and brain homogenate protein binding was determined *in vitro* using equilibrium dialysis, as described elsewhere [Zamek-Gliszczynski et al., J Pharm Sci, 101:1932-1940, 2012]. The results are reported as fraction unbound in plasma ($f_{u,plasma}$) and brain ($f_{u,brain}$) which are then utilized to calculate $K_{p,uu,brain}$ as described below. Mouse $f_{u,plasma}$ and $f_{u,brain}$ of Example 1 was determined to be 0.0667 and 0.011, respectively.

*Analysis and Results:* $K_{p,uu,brain}$ was calculated for each time point from the expression below where individual components are derived from a combination of *in vitro* and *in vivo* measurements carried out as described above:

$$K_{p,uu,brain} = \frac{C_{u,brain}}{C_{u,plasma}} = \frac{C_{total,brain}}{C_{total,plasma}} \cdot \frac{f_{u,brain}}{f_{u,plasma}}$$

where $C_{total,brain}$, $C_{u,brain}$, $C_{total,plasma}$, and $C_{u,plasma}$ are total and unbound brain and plasma concentrations, and $f_{u,brain}$ and $f_{u,plasma}$ are fractions unbound in brain and plasma, respectively.

**Table 2. Plasma and brain concentrations of Example 1 post 100 mg/kg oral dose in mouse.**

| Time point (Hours) | Dose Group (mg/kg) | Total brain conc. ($C_{total,brain}$) (nM) ±SD | Total plasma conc. ($C_{total,plasma}$) (nM) ±SD | Unbound brain conc. ($C_{u,brain}$) (nM) * ±SD | Unbound plasma conc. ($C_{u,plasma}$) (nM)^ ±SD | $K_{p,uu,brain}$ |
|---|---|---|---|---|---|---|
| 2 | 100 | 64100± 22700 | 18800±6260 | 706±250 | 1252±418 | 0.586±0.165 |
| *Using mouse $f_{u,brain}$ value of 0.011 and ^mouse $f_{u,plasma}$ value of 0.0667, as described above. | | | | | | |

Mean unbound brain to unbound plasma ratio (Kp,uu-brain) for the compound of Example 1 was 0.586, indicating that the compound has good penetration into the CNS and suggesting that an active transport mechanism is not operative in brain tissue in mouse.

**In vitro determination of intrinsic clearance in mice and human hepatocytes**

Cryopreserved hepatocytes are used to determine the in vitro metabolic clearance of pharmaceutical candidates. The following assay was conducted to compare the compound of Example 1 to its undeuterated analog, referred to hereinafter as Compound A:

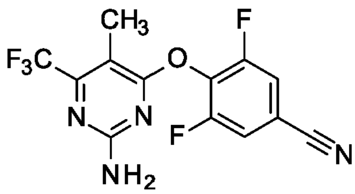

Compound A

Compound A is described in United States Patent No. 11,414,389, the entire contents of which are incorporated by reference herein.

To compare the metabolic clearance of the compound of Example 1 and Compound A, human and mouse cryopreserved hepatocytes were removed from liquid nitrogen storage, thawed in a 37°C water bath, placed in 50 mL Cryopreserved Hepatocyte Recovery Media (CHRM), centrifuged at 100 x g for 10 minutes for human and 65 x g for mouse, then re-suspended in Hepatocyte Maintenance Media (HMM). Compound incubations (0.3 μM substrate concentrations) were then performed in a 96-well plate format at 37°C with 200,000 cells/well, shaking at approximately 600 rpm. Incubations were initiated by direct addition of 2 μL of the substrate compounds. At 0, 15, 30, 60, and 90 minutes, 20 μL of incubation samples were quenched with 80 μL acetonitrile containing internal standard. Quenched plates were foil sealed, centrifuged for 30 minutes at 4000 rpm and analyzed for substrate by liquid chromatography with tandem mass spectrometry (LC-MS/MS).

The intrinsic clearance values of the compound of Example 1 and Compound A in mouse and human hepatocytes are shown in Table 3.

**Table 3. Human and mouse hepatocyte clearance**

| Hepatocyte intrinsic clearance | Compound of Example 1 | Compound A |
|---|---|---|
| Mouse (*u*L/min/*$10^6$cells)* | <1.80 | <1.80 |
| Human (*u*L/min/*$10^6$cells)* | 6.47 | 2.62 |

Intrinsic clearance is a measure of the maximum potential liver metabolism of a compound when not restricted by blood flow or protein binding This intrinsic clearance was determined from the elimination rate constants ($k_{dep}$; $min^{-1}$) measured from the disappearance of substrate in the incubations over time, using the following equation: $CL_{int}$ *($\mu$Lmin110$^6$cells) = $k_{dep}$ (min$^{-1}$) *0.2 mL incubated0.2 X 10$^6$cells *1000 $\mu$LmL*

**Determination of pharmacokinetics in mice**

*Subjects:* The subjects for these studies were male CD-1 mice from either Envigo, (Indianapolis, IN, USA) or Charles River Laboratories, Inc (Raleigh, NC, USA) between 4-12 weeks old at time of test. Each dosing group has three animals, and food and water is available *ad libitum.*

*Dosing and Sample Collection:* The test compound was administered intravenously (IV) via tail vein at 1 mg/kg (using vehicle: 25% (v/v) Dimethylacetamide (DMA), 15% (v/v) Ethanol (EtOH), 10% (v/v) Propylene Glycol (PG), 25% 2-Pyrrolidone(2-P), and 25% purified water) and orally (PO) via gavage needle at 10 mg/kg (using a vehicle of 1% (w/v) Hydroxyethylcellulose, 0.25% (w/v) Polysorbate 80, 0.05% (v/v) Antifoam1510-US in purified water *quantum satis.* Blood for dried blood spot (DBS) collection (approximately 20 µL) was collected from each available animal via a saphenous vein utilizing K2EDTA capillary tubes and stored at ambient temperature until analysis. Serial blood samples are collected at 0.08, 0.25, 0.5, 1, 2, 4, 8, 12, 24, and 48 h post dose for IV bolus and at 0.25, 0.5, 1, 2, 4, 8, 12, 24, and 48 h post dose after oral administration.

*Sample Analysis:* DBS cards (3 mm punches) of samples or standards were each mixed with a 180 uL solution of methanol:acetonitrile (1/1, v/v) containing internal standard to extract analyte followed by a two-fold dilution with water. A 10 uL aliquot of sample or standard is analyzed using an LC-MS/MS method. For Compound A, the lower limit of quantification (LLQ) was 5 ng/mL and the upper limit of quantification (ULQ) was 2500 ng/mL. For the compound of Example 1, the LLQ was 10 ng/mL and ULQ was 10,000 ng/mL

*Calculation of pharmacokinetic parameters:* Test article concentration data was uploaded into the Thermo Scientific™ Watson LIMS™ system where noncompartmental analysis was used to calculate Area Under the Curve (AUC) for both IV and PO arms, and Mean Residence Time (MRT) from the IV arm.

Bioavailability (%F) was calculated as follows,

$$\%F = (AUC_{PO} \text{ X } Dose_{IV}) / (AUC_{IV} \text{ X } Dose_{PO}) \text{ X } 100.$$

IV Clearance ($CL_{IV}$) was calculated as follows,

$$CL = Dose/AUC_{IV}$$

Volume of distribution at steady state (Vd,ss) was calculated as follows,

$$Vd,ss = CL*MRT$$

The pharmacokinetic parameters determined from the above-described assays are found in Table 4.

**Table 4. Pharmacokinetic parameters**

| PK Parameter | Compound of Example 1 | Compound A |
|---|---|---|
| $CL_{IV}$ (mL/min/kg) | 7.7 ± 1.3 | 16.1 ±1.7 |
| Vd,ss (L/kg) | 9.5 ± 3.1 | 6.6 ± 0.1 |
| %F | 60 ± 2.6 | 34 ± 3.7 |

The hepatocyte assay demonstrates that both the compound of Example 1 and Compound A have low metabolic turnover, but surprisingly in vivo studies in mice showed the compounds have a different pharmacokinetic profile, with the

compound of Example 1 exhibiting lower clearance and a higher oral bioavailability. These data suggest that despite similarly low intrinsic clearance, the deuterated compound of the present disclosure may allow for lower dose quantity and/or frequency while still achieving therapeutic levels of target engagement relative to Compound A.

**Claims**

1. A compound of the formula:

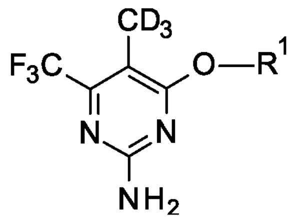

wherein $R^1$ is:

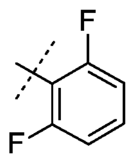
or
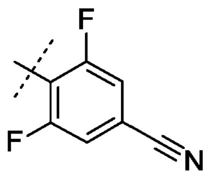

or a pharmaceutically acceptable salt thereof.

2. The compound according to claim 1 which is:

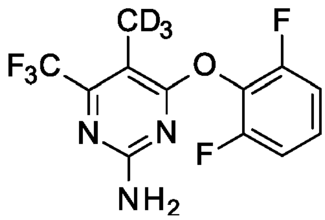

or a pharmaceutically acceptable salt thereof.

3. The compound according to claim 1 which is:

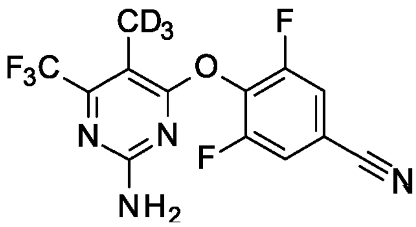

or a pharmaceutically acceptable salt thereof.

4. A compound according to claim 1 of the formula:

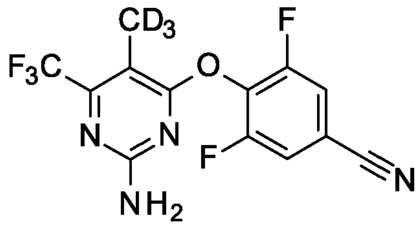

5. A compound according to claim 1 of the formula:

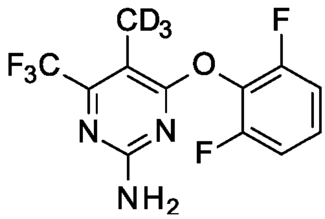

6. The compound or pharmaceutically acceptable salt thereof according to any one of claims 1 to 5 wherein each position represented as D has a deuterium enrichment of at least 50%.

7. The compound or pharmaceutically acceptable salt thereof according to any one of claims 1 to 5 wherein each position represented as D has a deuterium enrichment of at least 80%.

8. The compound or pharmaceutically acceptable salt thereof according to any one of claims 1 to 5 wherein each position represented as D has a deuterium enrichment of at least 90%.

9. The compound or pharmaceutically acceptable salt thereof according to any one of claims 1 to 5 wherein each position represented as D has a deuterium enrichment of at least 95%.

10. The compound or pharmaceutically acceptable salt thereof according to any one of claims 1 to 5 wherein each position represented as D has a deuterium enrichment of at least 99%.

11. A compound or pharmaceutically acceptable salt thereof according to any one of claims 1 to 10 for use in therapy.

12. A compound or pharmaceutically acceptable salt thereof according to any one of claims 1 to 10 for use in treating pain.

13. A compound or pharmaceutically acceptable salt thereof, according to any one of claims 1 to 10 for use in treating chronic pain.

14. A compound or pharmaceutically acceptable salt thereof, according to any one of claims 1 to 10 for use in treating chronic lower back pain.

15. A compound or pharmaceutically acceptable salt thereof, according to any one of claims 1 to 10 for use in treating diabetic peripheral neuropathic pain.

16. A compound or pharmaceutically acceptable salt thereof, according to any one of claims 1 to 10 for use in treating osteoarthritis pain.

17. A pharmaceutical composition, comprising a compound or a pharmaceutically acceptable salt thereof, according to any one of claims 1 to 10 with one or more pharmaceutically acceptable carriers, diluents, or excipients.

**Patentansprüche**

1. Verbindung der Formel:

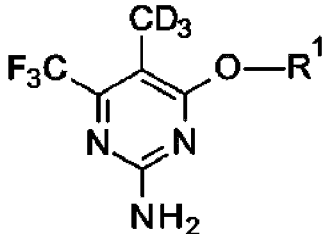

wobei $R^1$ ist:

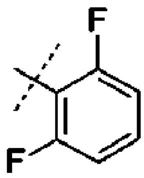 oder 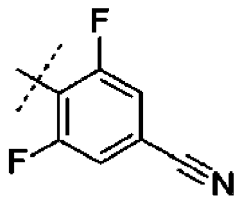

oder ein pharmazeutisch verträgliches Salz davon.

**2.** Verbindung nach Anspruch 1, die ist:

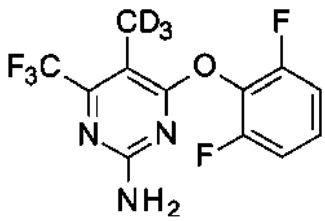

oder ein pharmazeutisch verträgliches Salz davon.

**3.** Verbindung nach Anspruch 1, die ist:

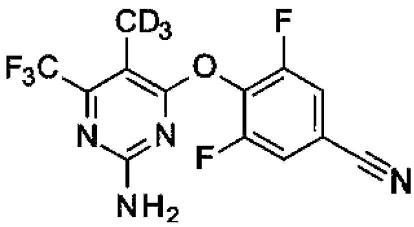

oder ein pharmazeutisch verträgliches Salz davon.

**4.** Verbindung nach Anspruch 1 der Formel:

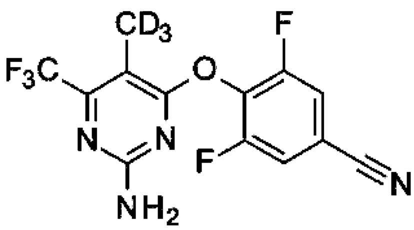

**5.** Verbindung nach Anspruch 1 der Formel:

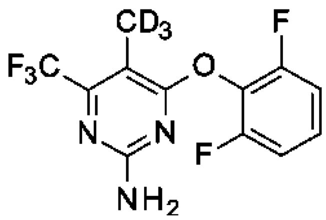

6. Verbindung oder pharmazeutisch verträgliches Salz davon nach einem der Ansprüche 1 bis 5, wobei jede als D dargestellte Position eine Deuteriumanreicherung von mindestens 50 % aufweist.

7. Verbindung oder pharmazeutisch verträgliches Salz davon nach einem der Ansprüche 1 bis 5, wobei jede als D dargestellte Position eine Deuteriumanreicherung von mindestens 80 % aufweist.

8. Verbindung oder pharmazeutisch verträgliches Salz davon nach einem der Ansprüche 1 bis 5, wobei jede als D dargestellte Position eine Deuteriumanreicherung von mindestens 90 % aufweist.

9. Verbindung oder pharmazeutisch verträgliches Salz davon nach einem der Ansprüche 1 bis 5, wobei jede als D dargestellte Position eine Deuteriumanreicherung von mindestens 95 % aufweist.

10. Verbindung oder pharmazeutisch verträgliches Salz davon nach einem der Ansprüche 1 bis 5, wobei jede als D dargestellte Position eine Deuteriumanreicherung von mindestens 99 % aufweist.

11. Verbindung oder pharmazeutisch verträgliches Salz davon nach einem der Ansprüche 1 bis 10 zur Verwendung in einer Therapie.

12. Verbindung oder pharmazeutisch verträgliches Salz davon nach einem der Ansprüche 1 bis 10 zur Verwendung bei einem Behandeln von Schmerzen.

13. Verbindung oder pharmazeutisch verträgliches Salz davon nach einem der Ansprüche 1 bis 10 zur Verwendung bei dem Behandeln chronischer Schmerzen.

14. Verbindung oder pharmazeutisch verträgliches Salz davon nach einem der Ansprüche 1 bis 10 zur Verwendung bei dem Behandeln chronischer Rückenschmerzen.

15. Verbindung oder pharmazeutisch verträgliches Salz davon nach einem der Ansprüche 1 bis 10 zur Verwendung bei dem Behandeln von diabetischen peripheren neuropathischen Schmerzen.

16. Verbindung oder pharmazeutisch verträgliches Salz davon nach einem der Ansprüche 1 bis 10 zur Verwendung bei dem Behandeln von Schmerzen durch Osteoarthritis.

17. Pharmazeutische Zusammensetzung, umfassend eine Verbindung oder ein pharmazeutisch verträgliches Salz davon nach einem der Ansprüche 1 bis 10 mit einem oder mehreren pharmazeutisch verträglichen Trägern, Verdünnungsmitteln oder Hilfsstoffen.

## Revendications

1. Composé de formule :

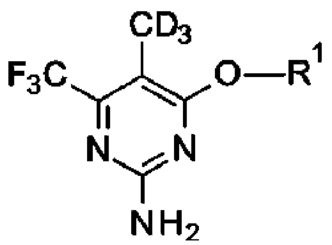

**où** $R^1$ est :

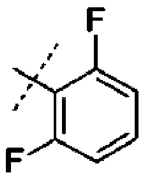 ou 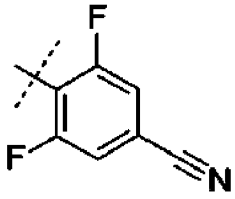

ou un sel pharmaceutiquement acceptable de celui-ci.

2. Composé selon la revendication 1 qui est :

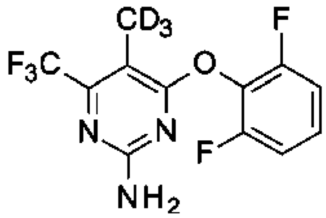

ou un sel pharmaceutiquement acceptable de celui-ci.

3. Composé selon la revendication 1 qui est :

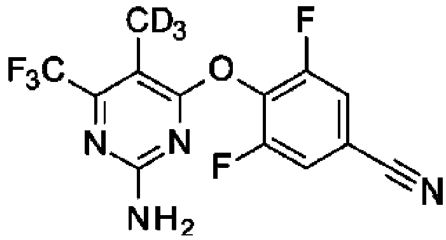

ou un sel pharmaceutiquement acceptable de celui-ci.

4. Composé selon la revendication 1 de formule :

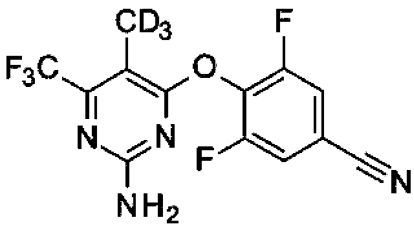

5. Composé selon la revendication 1 de formule :

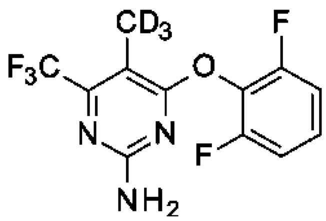

6. Composé ou sel pharmaceutiquement acceptable de celui-ci selon l'une quelconque des revendications 1 à 5, dans lequel chaque position représentée par D a un enrichissement en deutérium d'au moins 50 %.

7. Composé ou sel pharmaceutiquement acceptable de celui-ci selon l'une quelconque des revendications 1 à 5, dans lequel chaque position représentée par D a un enrichissement en deutérium d'au moins 80 %.

8. Composé ou sel pharmaceutiquement acceptable de celui-ci selon l'une quelconque des revendications 1 à 5, dans lequel chaque position représentée par D a un enrichissement en deutérium d'au moins 90 %.

9. Composé ou sel pharmaceutiquement acceptable de celui-ci selon l'une quelconque des revendications 1 à 5, dans lequel chaque position représentée par D a un enrichissement en deutérium d'au moins 95 %.

10. Composé ou sel pharmaceutiquement acceptable de celui-ci selon l'une quelconque des revendications 1 à 5, dans lequel chaque position représentée par D a un enrichissement en deutérium d'au moins 99 %.

11. Composé ou sel pharmaceutiquement acceptable de celui-ci selon l'une quelconque des revendications 1 à 10, pour une utilisation en thérapie.

12. Composé ou sel pharmaceutiquement acceptable de celui-ci selon l'une quelconque des revendications 1 à 10, pour une utilisation dans le traitement de la douleur.

13. Composé ou sel pharmaceutiquement acceptable de celui-ci selon l'une quelconque des revendications 1 à 10, pour une utilisation dans le traitement de la douleur chronique.

14. Composé ou sel pharmaceutiquement acceptable de celui-ci selon l'une quelconque des revendications 1 à 10, pour une utilisation dans le traitement de le traitement de la douleur lombaire chronique.

15. Composé ou sel pharmaceutiquement acceptable de celui-ci selon l'une quelconque des revendications 1 à 10, pour une utilisation dans le traitement de la douleur neuropathique périphérique diabétique.

16. Composé ou sel pharmaceutiquement acceptable de celui-ci selon l'une quelconque des revendications 1 à 10, pour une utilisation dans le traitement de la douleur liée à l'arthrose.

17. Composition pharmaceutique, comprenant un composé ou un sel pharmaceutiquement acceptable de celui-ci, selon l'une quelconque des revendications 1 à 10 avec un ou plusieurs supports, diluants ou excipients pharmaceutiquement acceptables.

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- US 6326368 B
- US 5100459 A
- US 11414389 B
- WO 2008052072 A


### Non-patent literature cited in the description


- **W. WANGDONG**. *ChemMedChem*, 2015, vol. 10 (1), 57-61
- Remington: The Science and Practice of Pharmacy. Elsevier Science, 2020
- **GOULD, P.L**. Salt selection for basic drugs. *International Journal of Pharmaceutics*, 1986, vol. 33, 201-217
- **BASTIN, R.J. et al.** Salt Selection and Optimization Procedures for Pharmaceutical New Chemical Entities. *Organic Process Research and Development*, 2000, vol. 4, 427-435
- **BERGE, S.M. et al.** , "Pharmaceutical Salts. *Journal of Pharmaceutical Sciences*, 1977, vol. 66, 1-19
- *Angew. Chem. Int. Ed.*, 2015, vol. 54, 5488-5492
- **ZAMEK-GLISZCZYNSKI et al.** *J Pharm Sci*, 2012, vol. 101, 1932-1940